# EUROPEAN PATENT APPLICATION

(11) **EP 0 922 455 A1**
(43) Date of publication of application: **16.06.1999**
(21) Application number: 98919534.2
(22) Date of filing: 11.05.1998
(51) Int. Cl.: A61K 31/195, A61K 31/215, A61K 38/08, A61K 38/10, A61K 38/57, C07K 14/16, C12N 9/99

(54) **HIV-1 PROTEASE INHIBITORS, PROCESS FOR PRODUCING THE SAME, AND MEDICINAL COMPOSITIONS**

(30) Priority: 14.05.1997 JP 13937297
(71) Applicant: NISSUI PHARMACEUTICAL CO., LTD., Toshima-ku, Tokyo 170-0002 (JP)
(72) Inventor: SHOUJI, Shouzou, Kumamoto 862-8003 (JP)
(74) Representative: Wächtershäuser, Günter, Prof. Dr.
(86) International application number: JP9802059
(87) International publication number: WO9851295

(57) **Abstract**

An inhibitor of HIV-1 protease comprising, as an effective component, a P2 peptide, which is a peptide obtained by translating genetic information on the HIV-1 virus. The inhibitor comprises, as an effective comserved component, a synthesis peptide having the same amino acid sequence as that of the P2 peptide, or an analogous compound to the P2 compound. The inhibitor of HIV-1 protease of the present invention is basically a compound containing methionine, and may specifically be a peptide having an amino acid sequence comprising Ala-Glu-Ala-Met-Ser-Gln-Val-Thr-Asn-Thr-Ala-Thr-Ile-Met.

## Description

### FIELD OF THE INVENTION

The present invention relates to an inhibitor of HIV-1 protease comprising, as an effective component, a peptide obtained by translating genetic information on the HIV-1 virus, and an analogue; a method for preparing the same; and a pharmaceutical composition.

### BACKGROUND OF THE INVENTION

Therapeutic agents of AIDS are agents causing stop of any one of the stages of the proliferation process of the HIV virus which is infectious and existing in lymphocytes. Such therapeutic agents of AIDS which have been made practicable in recent years are, for example, inhibitors of reverse transcriptase, or inhibitors of HIV protease. It is known that the inhibitors of HIV protease among these suppress the proliferation of the HIV virus by the following function.

When the HIV virus infects lymphocytes, the HIV virus causes the production of a protein precursor called Pr 55 or a fusion protein called P160 ^{gag-pol}, so that the HIV virus starts proliferating. These proteins are processed by HIV protease, to turn functional proteins having various functions such as P17, P24, HIV protease and reverse transcriptase, that is, virus parts. The inhibitor of HIV protease has a function of inhibiting the effect of this HIV protease, so as to obstruct maturity of the HIV virus. At present, the inhibitor of HIV protease has been expected as the most promising therapeutic agent of AIDS.

It is known that the inhibitors of HIV-1 protease effective for inhibiting HIV-1 protease can be roughly grouped into the following 4 types, in accordance with difference of inhibiting mechanism.

### I) Substrate-based protease inhibitor

The substrate-based protease inhibitor interacts three-dimensionally with aspartic acid which is an active center of HIV-1 to inhibit HIV-1 protease.

### II) Symmetry-based protease inhibitor

As a result of paying attention to a C2 symmetry structure in an active region of HIV-1 protease, which is a characteristic of HIV-1 protease, this inhibitor has a structure matching the symmetry structure.

### III) Nonpeptic protease inhibitor

Peptic agents are in general poor in oral absorption, and are insufficient in a working period, resulting in drawbacks that they cannot pass through the blood-brain barrier. Thus, the nonpeptic protease inhibitor is a nonpeptic inhibitor of HIV-1 protease developed for overcoming these drawbacks. Specifically, AG-1343 and VX-478 are known.

### IV) Inherent dimerization inhibitor

This is an inhibitor for inhibiting formation of a dimer which is an active body of HIV-1 protease. As this inhibitor, inhibitors designed on the basis of a peptide (Thr-Leu-Asn-Phe) basically corresponding to the C-terminal the amino acid sequence of HIV-1 protease were reported in, for example, J. Bio. Chem. 266: 15591-4 (1991) by Z. Y. Zhang et al. and Biochem. Biophys. Res. Commun. 179: 847 - 51 (1991) and Biochem. Biophys. Res. Commun. 184: 980 - 5 (1992) by H. J. Schramm et al. Besides, according to a report in Biochem. Biophys. Res. Commun. 194: 595 - 600 (19) by H. J. Schramm et al., they found that, from computer modeling and research of inhibitors of HIV-1 protease originating from the terminal amino acid segment of HIV-1 protease, finally Ac-TVSFNF has inhibitory activity, and this substance corresponds to the C-terminal sequence of p6 of HIV-1 and, therefore, p6 of HIV-1 functions as a dimerization inhibitor. Furthermore, with regard to an inhibitor for exhibiting a synergetic effect of inhibiting formation of a dimer and inhibiting an active region, Biochem. Biophys. Res. Commun. 222: 38 - 43 (1996) by T. Uhlikova et al. reported another inhibitor in which an inhibitor of dimerization and an inhibitor of an active region are combined.

An object of the present invention is to find a new inhibitor of HIV-1 protease which belongs to inherent dimerization inhibitors and is different from conventional inhibitors, and provide an inhibitory agent of HIV-1 protease, comprising this inhibitor as an effective component, a method for preparing the same, and a pharmaceutical composition.

### DISCLOSURE OF THE INVENTION

The present invention which is a base for solving the aforementioned problems is an inhibitor of HIV-1 protease comprising, as an effective component, a P2 peptide, which is a peptide resulting from translating genetic information on the HIV-1 virus, and an analogue based on an amino acid sequence of the P2 peptide. The effective component of the inhibitor of HIV-1 protease of the present invention can be prepared by amino acid synthesis of the same peptide as the peptide resulting from translating genetic information on the HIV-1 virus in an ordinary manner.

The inhibitor of HIV-1 protease of the present invention comprises, as an effective conserved component, a peptide containing in its amino acid sequence at least Ala-Glu-Ala-Met-Ser-Gln (sequence number 1).

A preferred embodiment of the inhibitor of HIV-1 protease of the present invention comprises, as an effective component, a peptide comprising Ala-Glu-Ala-Met-Ser-Gln-Val-Thr-Asn-Thr-Ala-Thr-Ile-Met (sequence number 2). The peptide as an effective component of the inhibitor of HIV-1 protease of the present invention may be modified by a substituent and/or an adduct or be formed into salt.

The inventors paid attention to the P2 region in the gag gene of the HIV-1 virus, found an amino acid sequence resulting from translating the genetic information thereon, prepared a peptide having this sequence by chemical synthesis, and experimented inhibition of HIV-1 protease by this peptide.

More specifically, the inventors paid attention to P2 peptide comprising an amino acid sequence: Ala-Glu-Ala-Met-Ser-Gln-Val-Thr-Asn-Thr-Ala-Thr-Ile-Met (abbreviation: AEAMSQVTNTATIM) (sequence number 2) resulting from translating the P2 region in the gag gene of a BH10 strain of the HIV-1 virus, found that the P2 peptide produced by the HIV-1 inhibits HIV-1 protease necessary for proliferation of the HIV-1 itself suicidally, and then has completed the present invention. The BH 10 strain of the HIV-1 virus is deposited as H9/HTLV-IIIB ATCC CRL-8543.

The P2 peptide is a peptide obtained by processing Pr55 ^{gag}, which is a gene product from the gag gene of an HIV-1 patient, by HIV-1 protease. In the inhibitor of HIV-1 protease of the present invention comprising the P2 peptide as an effective component, the gene product from genetic information on HIV-1 inhibits the proliferation mechanism of HIV-1 itself. Therefore, it can be mentioned that the P2 peptide is a suicidal inhibitor of the protease. Besides, the P2 peptide is a peptide which is inherent in the genetic information on HIV-1. Thus, it can be mentioned that the P2 peptide is an inherent inhibitor of the protease.

HPLC analysis has proved that the inhibiting mechanism of the inhibitor of HIV-1 of the present invention is based on inhibition of formation of the active dimer.

As shown in Table 1 below, however, the P2 peptide has an amino acid sequence commonly among individual strains of the HIV-1 virus (i.e., AEAMSQ), that is, a peptide comprising the peptide of the sequence number 1, and an amino acid sequence which continues to the peptide of the sequence number 1 and is somewhat different in the individual strains. Therefore, the inhibitor of HIV-1 protease of the present invention is not limited to the P2 peptide of the sequence number 2. Thus, the inhibitor may comprise, as an effective component, a peptide comprising at least Ala-Glu-Ala-Met-Ser-Gln (sequence number 1).

**Table 1**

| HIV-1 straim | Sequemce of P2 peptitde | Site on the gag |
|---|---|---|
| MN | AEAMSQ-VTNSATIM | 366-379 |
| ARV2/SF2 | AEAMSQ-VTNPANIM | 365-378 |
| BH10 | AEAMSQ-VTNTATIM | 363-376 |
| BH5 | AEAMSQ-VTNSTTIM | 363-376 |
| BRU | AEAMSQ-VTNSATIM | 363-376 |
| CDC-451 | AEAMSQ-VTNSATIM | 363-376 |
| ELI | AEAMSQ-ATNSVTTAM | 363-377 |
| HXB2 | AEAMSQ-VTNSATIM | 363-376 |
| JH3 | AEAMSQ-VTNSTTIM | 363-376 |
| JRCSF | AEAMSQ-VTNPATIM | 363-376 |
| MAL | AEAMSQ-ATNSTAAIM | 369-383 |
| NEW YORK-5 | AEAMSQ-VTNPATIM | 363-376 |
| NDK | AEAMSQ-VTGSATAVM | 360-374 |
| OYI | AEAMSQ-VNSVTVMM | 363-376 |
| PV22 | AEAMSQ-VTNTATIM | 363-376 |
| RF/HAT | AEAMSQ-VTNSATIM | 363-376 |
| U455 | AEAMSQ-VQQTSIM | 359-371 |
| WMJ2 | AEAMSQ-VTNPTTIM | 363-376 |
| YU2 | AEAMSQ-VTNSATIMM | 363-377 |
| Z2/CDC-Z34 | AEAMSQ-ATNSAAAVM | 364-378 |

The mechanism of inhibiting HIV-1 protease by the P2 peptide of the present invention is that formation of a dimer which is an active body of HIV-1 protease is inhibited (inherent dimerization inhibition), thus causing the inhibition of HIV-1 protease.

On the basis of the finding of the inhibitory activity of the respective peptides having the aforementioned sequence numbers 1 and 2 against HIV-1 protease, various peptides analogous to the sequence number 1 and the sequence number 2, and other compounds are synthesized by peptide synthesis and chemical synthesis, and then the inhibitory activity of these compounds against HIV-1 protease was examined. As a result, the inventors have found that compounds obtained by the following chemical synthesis have inhibitory activity against HIV-1 protease.

That is, a second aspect of the present invention is characterized by an inhibitor of HIV-1 protease which comprises at least an amino acid sequence comprising Ala-Glu-Ala-Met-Ser-Gln, which is equal to the sequence number 1, and is other peptide than the peptide sequence of the sequence number 2; and is the following peptide:
Ala-Glu-Ala-Met-Ser-Gln-Val-Thr-Asn-Ser-Ala-Thr-Ile-Met;
Ala-Glu-Ala-Met-Ser-Gln-Val-Ala-Asn-Thr-Ala-Thr-Ile-Met;
Ala-Glu-Ala-Met-Ser-Gln-Val-Thr-Asn; and
Ala-Glu-Ala-Met-Ser-Gln-Val-Thr-Leu.

An inhibitor of HIV-1 protease which comprises, as an effective component, various peptides analogous to the sequence number 1 and the sequence number 2, and other compounds, and has an inhibitory activity against HIV-1 protease is as follows:
an inhibitor of HIV-1 protease comprising, as an effective conserved component, a peptide containing an amino acid sequence comprising at least Ala-Glu-Ala-Met-Ala-Gln, and more specifically its peptide sequence is Ala-Glu-Ala-Met-Ala-Gln-Val-Thr-Asn.

Still another inhibitor of HIV-1 protease of the present invention is an inhibitor of HIV-1 protease comprising, as an effective conserved component, a peptide containing an amino acid sequence comprising at least Ala-Glu-Ala-Ala-Ser-Gln, and more specifically its peptide sequence is Ala-Glu-Ala-Ala-Ser-Gln-Val-Thr-Asn-Thr-Ala-Thr-Ile-Met.

Other inhibitor of HIV-1 protease of the present invention is an inhibitor of HIV-1 protease comprising, as an effective component, a peptide containing an amino acid sequence comprising at least Ala-Ala-Ala-Met-Ser-Gln, and more specifically its peptide sequence is Ala-Ala-Ala-Met-Ser-Gln-Val-Thr-Asn.

The inventors proceeded with investigation on the basis of amino acid structures of the peptides of the compounds having HIV-1 protease activity, further prepared various compounds, and studied the HIV-1 protease activity of these compounds. As a result, the following invention has been made.

That is, the present invention is an inhibitor of HIV-1 protease comprising a methionine residue-containing compound as an effective component. Preferred embodiments of the methionine residue-containing compounds include compounds having HIV-1 protease activity in the following groups:
group (1): compounds containing a methionine residue represented by the following formula (I): 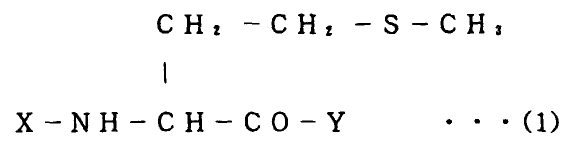 wherein X represents H, a residue of myristic acid, retinoic acid, geranic acid, geranylgeranic acid , β-carotene derivatives, capsaicin derivatives, or isoprenoid compounds, and Y represents OH or a residue of fatty acids having 1 - 20 carbon atoms;
group (2): Myr-Ala-Glu-Ala-Met, wherein Myr represents a residue of myristic acid bonded to the N-terminal; and
group (3): Myr-Ala-Glu-Ala-Met-Ser-Gln, wherein Myr represents a residue of myristic acid bonded to the N-terminal.

Preferred embodiments of the methionine residue-containing compounds represented by the formula (I) include compounds having HIV-1 protease activity in the following groups:
(1) a residue of myristic acid - a methionine residue (abbreviation: Myr-Met, wherein Myr represents a residue of myristic acid bonded to the N-terminal);
(2) a residue of fatty acids having 6 - 12 carbon atoms - a methionine residue (abbreviation: C₆₋₁₂ - Met);
(3) a residue of lauric acid - a methionine residue (abbreviation: Lau-Met, wherein Lau represents a residue of lauric acid bonded to the C-terminal); and
(4) a residue of palmitic acid - a methionine residue (abbreviation: Pal - Met, wherein Pal represents a residue of palmitic acid bonded to the C-terminal.

In the case wherein the N-terminal in the compound represented by the formula (I) is an isoprenoid compound, it is preferred that the compound is in a trans-form. For example, eicosapentaenoic acid or docosahexaenoic acid is preferred.

The various aforementioned types of peptides having inhibitory activity against HIV-1 protease and the various aforementioned types of compounds having inhibitory activity, according to the present invention, way be modified by a substituent and/or an adduct, or may be made into a pharmacologically acceptable salt. Examples of the pharmacologically acceptable salt include salts of hydrochloric acid, sulfuric acid, nitric acid, nitrous acid, hydrobromic acid, hydroiodic acid, phosphoric acid, and organic acids.

The inhibitor of HIV-1 protease and the pharmaceutical composition comprising as an effective component the P2 peptide, of the present invention, are used in an oral agent or a parenteral agent form. Examples of the oral agent form include forms of a tablet, a pulvis (powder), a granular agent, a capsule agent, a microcapsule agent and a liquid agent. The parenteral agent is used in a form of a liquid agent, and mainly in a form of an injection or a suppository. It is in general acceptable to incorporate, into these medicines, well-known auxiliary components for preparing medicines such as an excipient, a binder, a disintegrating agent, a lubricant, a stabilizer, and a flavor.

The dose of thereof varies dependently on the conditions of patients and their ages. In case of oral administration, for normal adults an amount of 0.1 - 1000 mg per kg of their weight can be administered every day.

### BRIEF DESCRIPTION OF THE DRAWINGS

Figure 1 is a graph showing results obtained by calculating initial velocities of reaction by HIV-1 protease used in the present invention at various substrate concentrations and then analyzing them from Lineweaver-Burk plots.
Figure 2 is a graph showing the relative activity of the peptide of the sequence number 2 at its various concentrations, the relative activity being represented as a percentage relative to the enzyme activity (value: 100) in the absence of the peptide of the sequence number 2.
Figure 3 is a graph showing results obtained by calculating initial velocities of reaction by HIV-1 protease at various substrate concentrations, in the presence of the peptide of the sequence number 2, and then analyzing them from Lineweaver-Burk plots.
Figure 4 is a graph showing a result obtained by analyzing Ala-Glu-Ala-Met-Ser-Gln-Val-Thr-Asn-Thr-Ala-Thr-Ile-Met, which is an effective component of the inhibitor of HIV-1 protease of the present invention, by means of a laser ionizing the mass spectrometer.

### BEST MODES FOR CARRYING OUT THE INVENTION

### Example 1

### Preparation of HIV-1 protease

Plasmid pLB550-1 for expressing a fusion protein of partial pol protein containing HIV-1 protease and β-galactosidase was transfected into E. coli K12 (UT481) [Werman, K. F. et al. J. Bacteriol, 163: 376 - 384 (1985)] by the CaCl₂ method, and was enzyme-induced by isopropyl-β-D(-)-thiogalactopyranoside and was cultured for 6 hours. Thereafter, the bacteria were collected. The bacterial were treated with lysozyme and crashed with glass beads. Subsequently, the solubilized supernatant was purified by DEAE-cellulofine, SP-Toyopearl and pepstatin A agarose column chromatography, to obtain a purified HIV-1 protease.

### Decision of a catalytic parameter

### 1) Km and Vmax

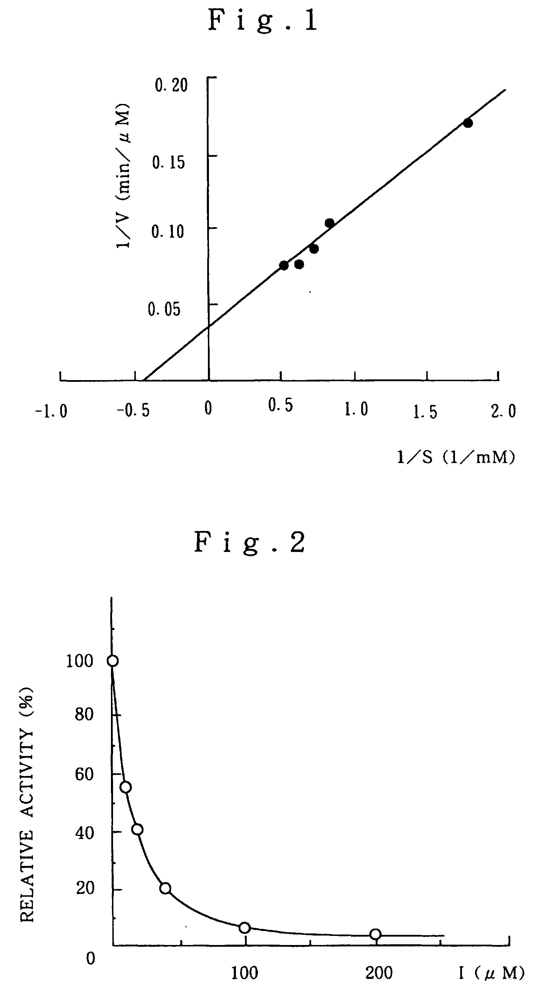

Synthesized peptide composed of Suc-Ser-Gln-Asn-Tyr-Pro-Ile-Val-Gln as a substrate was dissolved into 50 mM MES-NaOH buffer solution (pH 5.5) so that its concentration in enzyme reaction would be 0.57 mM, 1.25 mM, 1.43 mM, 1.67 mM and 2.0 mM. Into 50 µl of these substrate solutions, 50 µl of the purified enzyme solution of HIV-1 protease, obtained in the aforementioned preparing step, was added and then enzyme reaction was carried out at 37°C for 0 , 5, 10 and 15 minutes, respectively. To these solutions, 11.1 µl of acetonitryl containing 1% TFA was added to stop the enzyme reaction. The peptides obtained by the enzyme reaction were separated by high-performance liquid chromatography. Thereafter, enzyme activity thereof was measured by means of a PICO-TAG amino acid composition analyzing device. The initial velocities at the respective concentrations of the substrate were calculated and then analysis was performed based on by Lineweaver-Burk plots. A graph showing the results is shown in Fig. 1. Km obtained from the graph was 2.2 mM, and Vmax was 27.5 µM/min. In the substrate, the cleavage region was between Tyr and Pro.

### 2) IC₅₀

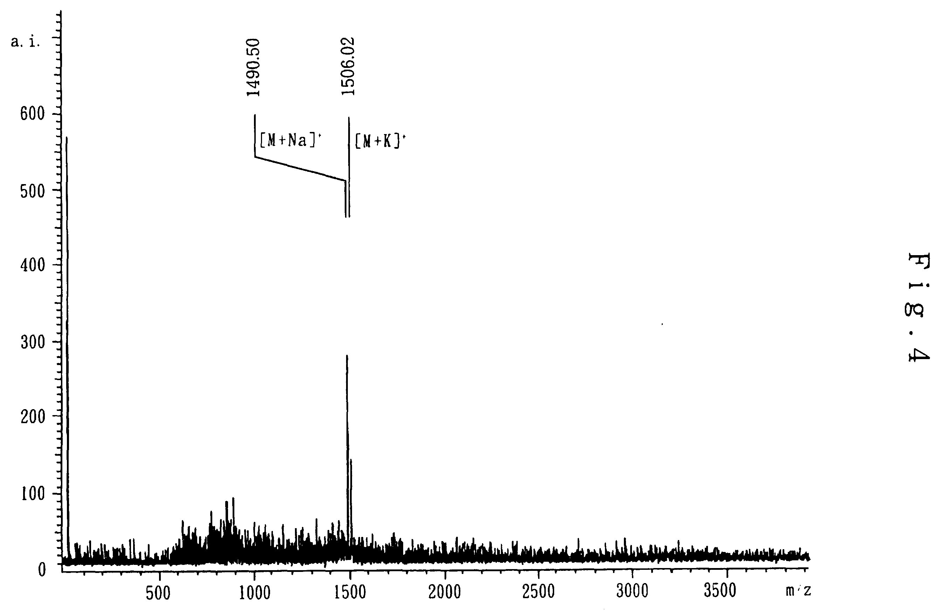

According to Nature 313: 277 - 284 (1985) by Ratner, L. et al., the peptide sequence in the HIV-1 BH 10 strain is a peptide positioned between 363 and 376 sites of the gag protein, and is Ala-Glu-Ala-Met-Ser-Gln-Val-Thr-Asn-Thr-Ala-Thr-Ile-Met (abbreviation: AEAMSQVTNTATIM) (the sequence number 2). The peptide of the sequence number 2 was synthesized in a peptide synthesizing device in an ordinary manner. Specifically, by solid phase synthesis (J. Am. Chem. Soc., 85, 2149 (1963) by R. B. Merrifield, the peptide was synthesized on a resin as a solid phase from methionine (M: the first amino acid) to a final amino acid, i.e., alanine (A: the fourteenth amino acid), in turn, and then the peptide was cleaved from the resin by trifluoroacetic acid and was taken out. The peptide was purified to obtain a peptide of the sequence number 2 equal to the aforementioned sequence. The average molecular weight of the obtained peptide of the sequence number 2 was 1468.6948. Figure 4 is a graph shoving a result obtained by subjecting the peptide of the sequence number 2 to laser ionizing mass spectroscopy.

On the other hand, the synthesized peptide, Suc-Ser-Gln-Asn-Tyr-Pro-Ile-Val-Gln as a substrate was used to measure inhibitory activity of the peptide of the sequence number 1 against HIV-1 protease as follows: 50 µl of the enzyme solution was added to 30 µl of solution of the peptide of the sequence number 1 in which the peptide was dissolved into 50 mM of MES-NaOH buffer solution (pH 5.5) so that its final concentration would be 10, 20, 40, 100 and 200 µM, and then enzyme reaction was carried out at 37 °C for 30 minutes.

Next, the solution of the substrate, Suc-Ser-Gln-Asn-Tyr-Pro-Ile-Val-Gln was added thereto so that its final concentration would be 1 mM, and then enzyme reaction was carried out at 37 °C for 0, 5, 10 and 15 minutes. To these solutions, 11.1 µl of acetonitryl containing 1% TFA was added to stop the enzyme reaction. The peptides obtained by the enzyme reaction were separated by high-performance liquid chromatography. Thereafter, enzyme activity thereof was measured by means of a PICO-TAG amino acid composition analyzing device. Figure 2 shows, in a graph, the relative activity of the peptide of the sequence number 2 at its respective concentrations. (The relative activity is represented as a percentage relative to the enzyme activity (value: 100) in the absence of the peptide of the sequence number 2.) From this graph, IC₅₀ was calculated and obtained, so that IC₅₀ was 10 µM.

### 3) Ki

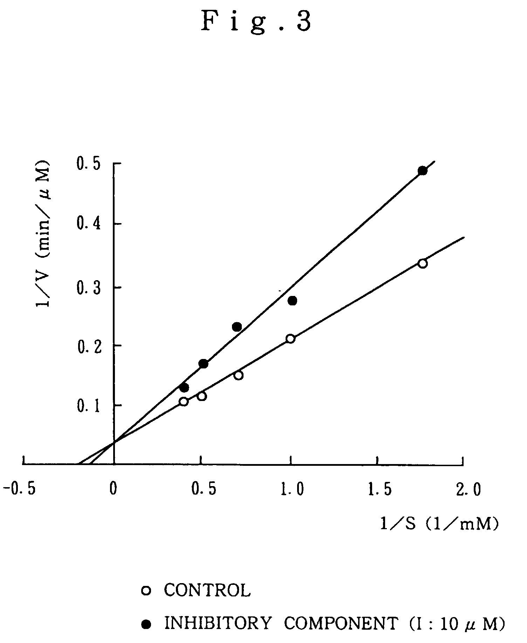

50 µl of the enzyme solution was added to 30 µl of the peptide of the sequence number 2 dissolved into 50 mM of MES-NaOH buffer solution so that its final concentration would be 10 µM and then enzyme reaction was carried out at 37 °C for 30 minutes. The solution of the substrate, Suc-Ser-Gln-Asn-Tyr-Pro-Ile-Val-Gln was added thereto so that its final concentration would be 0.579 1.0, 1.43, 2.0 and 2.5 mM, and then enzyme reaction was carried out at 37 °C for 0, 5, 10 and 15 minutes. To these solutions, 11.1 µl of acetonitryl containing 1% TFA was added to stop the enzyme reaction, and then analysis was performed from Lineweaver-Burk plots. The results are shown in Fig. 3 as a graph. Ki obtained from the graph was 30.2 µM. A control experiment was carried out in the absence of the peptide of the sequence number 2, in the same way.

### Decision of inhibitory mechanism

150 µl of P2 peptide dissolved into 50 mM MES (pH 5.5) buffer solution containing 100 mM NaCl was added to a fraction containing the inhibitor HIV-1 protease purified by the aforementioned method, that is, an SP-Toyopearl fraction (150 µl), so that its final concentration would be 30 µM, and subsequently the solution was incubated at 37 °C for 30 minutes. With respect to conditions at this time, the used column was TSK-G2000SW, AUFS was 0.02, PSI was 100, the chart rate was 0.5, the flow rate was ml/ml, the detector was of 280 nm length, and the discharged volume was 300 µl.

In a control experiment, only HIV-1 protease was subjected to the molecular sieve HPLC. In this case, a peak (24 minutes) corresponding to the dimer, which was an active body, was obtained. Addition of the peptide of the sequence number 2 (final concentration: 30 µM) caused decrease in the peak corresponding to the dimer and increase in a peak (26 minutes) corresponding to the monomer accordingly. Therefore, it was able to be understood that the inhibitory mechanism of the peptide of the sequence number 2 is based on inhibition of formation of the dimer of HIV-1 protease.

### Example 2

Thirty kinds of compounds 1 - 30 shown in Table 2 below were prepared in an ordinary manner. For example, N-Myr-AEAMSQ, or N-Myr-AEAM was prepared as follows. That is, 0.25 mg of AEAMSQ-resin or 0.25 mg of AEAM-resin was prepared, and 15 ml of CH₂Cl₂ containing 2 % TEA and 150 µl of myristoyl chloride was added thereto. This was then stirred for 5 hours, followed by washing by water and releasing from the resin to obtain N-Myr-AEAMSQ, or N-Myr-AEAM.

N-acyl-Met having 10 carbon atoms was obtained as follows. That is, 0.75 g of methionine was prepared, and thereto was added 15 ml of AcCNa, 10 ml of TEA and further 10 ml of acyl (C₁₀) chloride. This was stirred all night. Furthermore, 15 ml of deionized water was added thereto, and then stirred for 1 hour. Thereafter, ether was added to the resultant so that the ratio of the two would be 1 : 1, and then a middle layer was separated, purified, freeze-dried, to obtain N-acyl-Met having 10 carbon atoms.

Concerning these 30 kinds of compounds, inhibitory effect against of HIV protease and IC₅₀ were measured in the same way as in 2) in Example 1. The obtained results are shown in Table 2.

**Table 2**

| Compound | | IC50(µM) | Theoretical mass value [M+H]⁺ | Measured mass value [M+Na]⁺ | Measured mass value [M+K]⁺ |
|---|---|---|---|---|---|
| 1 | AEAMSQVTNTATIM | 10 | | | |
| 2 | AEAMSQVTNSATIM | 22 | | | |
| 3 | AEAMSQVTNPATIM | - | | | |
| 4 | AEAMSQVTNTATIM | + | | | |
| 5 | AEAMSQVANTATIM | + | | | |
| 6 | AEAMSQVTN | 6 | 950.4252 | 972.4252 | 988.4252 |
| 7 | AAAMSQVTN | + | | | |
| 8 | AEAASQVTN | - | | | |
| 9 | AEAMAQVTN | + | | | |
| 10 | AEAMSQVAN | - | | | |
| 11 | AEAMSQVTL | 53 | | | |
| 12 | AEAMSQVT | - | | | |
| 13 | AEAMSQV | - | | | |
| 14 | AEAMSQAoc | - | | | |
| 15 | AEAMSQAhx | - | | | |
| 16 | Suc-AEAMSQAhx | - | | | |
| 17 | AEAMSQ | - | | | |
| 18 | N-Myr-AEAMSQ | 79 | 846.4645 | 868.4645 | 884.4645 |
| 19 | AEAM | - | | | |
| 20 | N-Myr-AEAM | 16 | 631.3739 | 653.3739 | 669.3739 |
| 21 | VTN | - | | | |
| 22 | VTNTATIM | - | | | |
| 23 | N-Myr-M | 44 | 360.2571 | 382.2571 | 398.2571 |
| 24 | N-C₁₀-M | 179 | 304.1945 | 326.1945 | 342.1945 |
| 25 | N-Lau-M | 124 | 332.2258 | 354.2258 | 370.2258 |
| 26 | N-Pal-M | 98 | 388.2883 | 410.2883 | 426.2883 |
| 27 | N-AocM | - | | | |
| 28 | N-AocMAoc | - | | | |
| 29 | N-AocMAocAoc | - | | | |
| 30 | N-AocAocMAoc | - | | | |
| Aoc : 8-aminooctanoyl or 8-aminooctanoic acid Ahx : 6-aminohexanoic acid C₁₀ : decanoyl Lau : lauroyl Myr : myristoyl Pal : palmitoyl + : Inhibitory effect is exhibited at 500 µM or less. - : Inhibitory effect is not exhibited at 500 µM or less. | | | | | |

According to Table 2, it was recognized that the compounds Nos. 1, 2, 4-7, 9, 11, 18, 20 and 20-26 had activity against HIV protease.

### INDUSTRIAL AVAILABILITY

The P2 peptide obtained as a gene product of the HIV-1, according to the present invention, more specifically Ala-Glu-Ala-Met-Ser-Gln-Val-Thr-Asn-Thr-Ala-Thr-Ile-Met, has inhibitory activity against HIV-1 protease.

The substance having inhibitory activity against HIV-1 protease of the present invention belongs to inherent dimerization inhibitors, and is a new inhibitor of HIV-1 protease which is different from conventional inhibitors.

It is suggested that since the inhibitor of HIV-1 protease of the present invention is suicidal information originally recorded in genetic information on the HIV-1 virus, there is not a possibility of appearance of resistant strains, as observed in conventional therapeutic agents of AIDS.

## Claims

1. An inhibitor of HIV-1 protease, comprising a methionine residue-containing compound as an effective component.

2. The inhibitor of HIV-1 protease according to claim 1, wherein mechanism of inhibiting the HIV-1 protease is based on inhibiting the formation of a dimer of the HIV-1 protease, which is an active body of the HIV-1 protease.

3. The inhibitor of HIV-1 protease according to claim 1, wherein the methionine residue-containing compound is selected from the groups of the following 1) - 7):
1)compounds containing a methionine residue represented by the following formula (I): 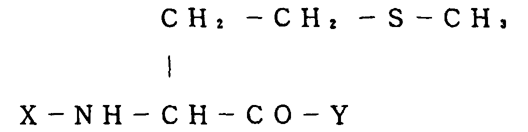 wherein X represents H, a residue of myristic acid, retinoic acid, geranic acid, geranylgeranic acid, β-carotene derivatives, capsaicin derivatives, or isoprenoid compounds, and Y represents OH, or a residue of fatty acids having 1 - 20 carbon atoms,
2) Myr-Ala-Glu-Ala-Met, wherein Myr represents a residue of myristic acid bonded to the N-terminal; and
3) Myr-Ala-Glu-Ala-Met-Ser-Gln, wherein Myr represents a residue of myristic acid bonded to the N-terminal.
4) a peptide comprising at least Ala-Glu-Ala-Met-Ser-Gln,
5) a peptide comprising at least Ala-Glu-Ala-Met-Ala-Gln,
6) Ala-Glu-Ala-Ala-Ser-Gln-Val-Thr-Asn-Thr-Ala-Thr-Ile-Met, and
7) a peptide comprising at least Ala-Ala-Ala-Met-Ser-Gln.

4. The inhibitor of HIV-1 protease according to claim 3, wherein the compound represented by the group 1) is selected from the following:
Myr-Met, wherein Myr represents a residue of myristic acid bonded to the N-terminal;
A residue of fatty acids having 6 - 12 carbon atoms - Met;
Lau-Met, wherein Lau represents a residue of lauric acid bonded to the C-terminal; and
Pal - Met, wherein Pal represents a residue of palmitic acid bonded to the C-terminal.

5. The inhibitor of HIV-1 protease according to claim 3, wherein the compound represented by the group 4) is selected from the following compounds i) - v):
i) Ala-Glu-Ala-Met-Ser-Gln-Val-Thr-Asn-Thr-Ala-Thr-Ile-Met;
ii) Ala-Glu-Ala-Met-Ser-Gln-Val-Thr-Asn-Ser-Ala-Thr-Ile-Met;
iii) Ala-Glu-Ala-Met-Ser-Gln-Val-Ala-Asn-Thr-Ala-Thr-Ile-Met;
iv) Ala-Glu-Ala-Met-Ser-Gln-Val-Thr-Asn; and
v) Ala-Glu-Ala-Met-Ser-Gln-Val-Thr-Leu.

6. The inhibitor of HIV-1 protease according to claim 3, wherein the compound represented by the group 5) is the following compound:
Ala-Glu-Ala-Met-Ala-Gln-Val-Thr-Asn.

7. The inhibitor of HIV-1 protease according to claim 3, wherein the compound represented by the group 7) is the following compound:
Ala-Ala-Ala-Met-Ser-Gln-Val-Thr-Asn.

8. The inhibitor of HIV-1 protease according to claim 1, 2, 3, 4, 5, 6 or 7, wherein the compound is modified by a substituent and/or an adduct, or may be formed into a salt.

9. An inhibitor of HIV-1 protease, which is a peptide resulting from translating genetic information of a HIV-1 virus, and comprises, as an effective component, a suicidal substance of inhibiting the HIV-1 protease.

10. The inhibitor of HIV-1 protease according to claim 9, wherein the suicidal substance is a peptide originating from the P2 peptide.

11. An inhibitor of HIV-1 protease, comprising, as an effective component, a peptide having the same amino acid sequence as that of the peptide according to claim 9 or 10, prepared by chemical synthesis.

12. A pharmaceutical composition, comprising a compound according to claim 1, 2, 3, 4, 5, 6, 7, 8, 9, 10 or 11 in a pharmacologically effective amount, and the compound is bonded to a pharmacologically acceptable salt.

13. A method for preparing an inhibitor of HIV-1 protease, comprising finding a suicidal substance for inhibiting the HIV-1 protease from gene products of any HIV virus, and separating and purifying the substance to obtain the inhibitor of the HIV-1 protease.
